# FASCICULE DE BREVET EUROPEEN

(11) **EP 2 874 999 B1**
(45) Date de publication et mention de la délivrance du brevet: **24.08.2016**
(21) Numéro de dépôt: 13747479.7
(22) Date de dépôt: 23.07.2013
(51) Int. Cl.: C07C 317/44, A61K 45/06, A61K 8/46, A61Q 19/08

(54) **NOUVEAUX COMPOSES INHIBITEURS SELECTIFS DU CYP26A1 UTILES DANS DES COMPOSITIONS COSMÉTIQUES ET PHARMACEUTIQUES**
NEUARTIGE SELEKTIVE VERBINDUNGEN ZUR CYP26A1-HEMMUNG, VERWENDBAR IN KOSMETISCHEN UND PHARMAZEUTISCHEN ZUSAMMENSETZUNGEN
NOVEL SELECTIVE COMPOUNDS INHIBITING CYP26A1 USEFUL IN COSMETIC AND PHARMACEUTICAL COMPOSITIONS

(30) Priorité: 23.07.2012 FR 1257128
(43) Date de publication de la demande: 27.05.2015
(73) Titulaire: Galderma Research & Development, 06410 Biot (FR)
(72) Inventeur: BOITEAU, Jean-Guy, F-06560 Valbonne (FR); RIVIER, Michel, F-06100 Nice (FR); FERAILLE, Gérard, F-06250 Mougins (FR); JOMARD, André, F-06460 Saint Vallier De Thiey (FR)
(74) Mandataire: Chajmowicz, Marion
(86) Numéro de dépôt international: PCT/FR2013/051769
(87) Numéro de publication internationale: WO 2014/016507

(56) Documents cités:
- WO-A1-99/10322
- NJAR V C O ET AL: "Retinoic acid metabolism blocking agents (RAMBAs) for treatment of cancer and dermatological diseases", BIOORGANIC & MEDICINAL CHEMISTRY, PERGAMON, GB, vol. 14, no. 13, 1 juillet 2006 (2006-07-01), pages 4323-4340, XP027992703, ISSN: 0968-0896 [extrait le 2006-07-01] cité dans la demande
- KUO-LONG Y ET AL: "Structural modifications of 6-naphthalene-2-carboxylate retinoids", BIOORGANIC & MEDICINAL CHEMISTRY LETTERS, PERGAMON, ELSEVIER SCIENCE, GB, vol. 6, no. 23, 3 décembre 1996 (1996-12-03), pages 2865-2870, XP004136095, ISSN: 0960-894X, DOI: 10.1016/S0960-894X(96)00524-0

## Description

La présente invention porte sur de nouveaux composés, sur les compositions pharmaceutiques contenant ces composés ainsi que sur l'utilisation de ces composés et de ces compositions pour le traitement de pathologies. Elle concerne également les utilisations cosmétiques des nouveaux composés.

La présente invention porte sur des nouveaux composés étant des inhibiteurs sélectifs de CYP26A1, leur utilisation et leur synthèse.

La vitamine A et ses dérivés, les rétinoïdes, sont impliqués dans divers processus biologiques, comme la prolifération cellulaire, la différenciation et l'apoptose.
Les rétinoïdes naturels et synthétiques sont classiquement utilisés dans le traitement des pathologies cutanées telles que l'acné, le psoriasis et l'ichthyose. (Van de Kerkhof PC. Dermatol. Ther. 2006; 19:252-63). Mais ces actifs peuvent également causer une grande variété d'effets indésirables, comme l'irritation de la peau (Mills CM, Marks R. Drug Saf 1993; 9:280-90.) ce qui a conduit les chercheurs à investiguer vers de nouveaux médicaments, qui avaient les mêmes effets positifs des rétinoïdes mais sans leurs effets secondaires.

Un autre objectif des chercheurs a été de cibler l'effet pharmacologique souhaité au niveau des tissus où la vitamine A est métabolisée.

L'acide rétinoïque (RA), qui est le métabolite actif de la vitamine A, existe sous 4 formes isomères, l'acide all-trans rétinoïque (atRA), l'acide 13-cis rétinoïque (13cisRA) l'acide 9-cis rétinoïque (9cisRA) et l'acide 9, 13-dicis rétinoique.
Après la distribution de vitamine A dans la peau par la retinol-binding protein sérique, les kératinocytes, soit stockent la vitamine sous forme d'esters de rétinol, par une réaction catalysée par l'enzyme lécithine rétinol acyltransférase (LRAT) ou la métabolisent en atRA par une réaction en deux étapes, dans laquelle il est catalysé par la retinal déshydrogénase-2 de façon irréversible (RALDH2; ALDH1A2) (Roos TC, Jugert FK, Merk HF et al. Pharmacol Rev 1998; 50:315-33). L'atRA est biologiquement le métabolite le plus actif et se lie à des facteurs de transcription nucléaires spécifiques, comme les récepteurs de l'acide rétinoïque (RARs), qui régulent l'expression de nombreux gènes.

La dégradation cellulaire de l'atRA est régulée par une famille d'enzymes microsomales cytochrome P450 (CYP)-dépendantes, incluant la CYP26. La famille CYP26 est composée de trois isoformes CYP26A1, CYP26B1 et CYP26C1, (Abu-Abed SS, Beckett BR, Chiba H et al. J Biol Chem 1998; 273:2409-15; Sonneveld E, van den Brink CE, van der Leede BM et al. Cell Growth Differ 1998; 9:629- 37; Ray WJ, Bain G, Yao M et al. J Biol Chem 1997; 272:18702-8; Taimi M, Helvig C, Wisniewski J et al. J Biol Chem 2004; 279:77-85). Les isoformes CYP26A1 et CYP26B1 oxydent l'atRA en acide 4-hydroxyrétinoïque et en acide 4-oxo-rétinoïque, beaucoup moins actifs (Duell EA, Astrôm A, Griffiths CE et al. J Clin Invest 1992; 90:1269-74). Le dernière isoforme, CYP26C1 semble jouer un rôle métabolique sensiblement différent en étant capable de métaboliser à la fois l'atRA mais également l'acide rétinoique 9-cis (Helvig C, Taimi M, Cameron D, Jones G, Petkovich M. J Pharmacol Toxicol Methods. 2011 Nov-Dec;64(3):258-63). Toutes ces enzymes CYP26 peuvent être induites par de l'atRA exogène provoquant l'induction de sa dégradation (Loudig O, Babichuk C, White J et al. Mol Endocrinol 2000; 14:1483-97). Les études sur les animaux dont le gène codant pour le CYP26A1, le CYP26B1 ou le CYP26C1 avait été inactivé (souris Knock-out ou KO) ont montré que les CYP26A1 et CYP26B1 sont des protéines essentielles (Thatcher JE, Isoherranen N. Expert Opin Drug Metab Toxicol. 2009 Aug;5(8):875-86.). En effet, les phénotypes Cyp26a1-/- et Cyp26b1-/-, (c'est-à-dire des phénotypes homozygotes n'exprimant pas le gène CYP26a1 ou b1) sont léthaux. Les animaux produits par recombinaison homologue ne sont pas viables, contrairement au phénotype Cyp26c1-/- qui lui ne montre pas d'altération au niveau du développement embryonnaire ou à la naissance. Le fait que les animaux KO CYP26a1 et b1 ne soient pas viables, souligne l'importance respective de ces 2 protéines et le fait qu'elles ne sont pas redondantes d'un point de vue fonctionnel : en effet, la perte de CYP26A1 n'est pas compensée par CYP26B1 et vice versa. En conséquence, elles ont des rôles très différents et doivent être considérés comme des entités distinctes. Des études d'expression des ARNm et des protéines pour ces 2 CYP26 ont mis en évidence un profil d'expression assez similaire, avec toutefois des différences marquées pour certains organes (Topletz AR, Thatcher JE, Zelter A, Lutz JD, Tay S, Nelson WL, Isoherranen N. Biochem Pharmacol. 2012 Jan 1;83(1):149-63). Par exemple, l'ARNm de l'isoforme CYP26A1 est fortement exprimé au niveau du foie chez l'homme alors que l'isoforme CYP26B1 y est quasiment absent. L'ARNm de l'isoforme CYP26A1 est au contraire non détecté dans la vessie, le colon et l'ileum, alors que l'ARNm du CYP26B1 y est présent. Inversement, CYP26B1 est très fortement exprimé dans le cervelet, alors que l'isoforme CYP26A1 n'y est pas détecté. Ces différences observées au niveau des ARNm sont en partie également retrouvées au niveau protéique et d'une manière générale, il apparait que l'isoforme CYP26B1 est exprimé de manière assez ubiquitaire, alors que l'isoforme CYP26A1 présente un profil d'expression plus restreint (Topletz AR, Thatcher JE, Zelter A, Lutz JD, Tay S, Nelson WL, Isoherranen N. Biochem Pharmacol. 2012 Jan 1;83(1):149-63). Compte tenu du fait que le CYP26B1 est exprimé dans un grand nombre de tissus et que son activité biologique semble distincte de celle du CYP26A1, il apparait clairement que des inhibiteurs spécifiques du sous-type CYP26A1 présenterait l'avantage majeur de ne bloquer les voie métaboliques de l'atRA que dans un nombre limité de tissus, en particulier la peau. Cela aurait pour conséquence de limiter les effets adverses potentiellement médiés par l'isoforme CYP26B1 et donc d'améliorer la sécurité des patients pouvant être traités par ce type d'inhibiteurs.

Ainsi, en inhibant sélectivement l'activité CYP26A1, il devrait être possible d'augmenter le niveau endogène de l'atRA dans la peau. Il a été démontré que l'activité enzymatique du CYP26A1 est très supérieure à celle du CYP26B1. En effet, l'activité enzymatique de déplétion en atRA du CYP26A1 est de l'ordre de 20 fois celle du CYP26B1. Dans la peau, les 2 isoformes étant exprimés de la même manière au niveau protéique, il est donc raisonnable de penser que l'isoforme CYP26A1 est majoritairement responsable de l'élimination de l'atRA (Topletz AR, Thatcher JE, Zelter A, Lutz JD, Tay S, Nelson WL, Isoherranen N. Biochem Pharmacol. 2012 Jan 1;83(1):149-63). Cela ne sera par contre pas le cas dans les tissus où le CYP26A1 n'est pas exprimé et où l'activité d'élimination de l'atRA sera assurée par le CYP26B1, comme le cervelet.
Un inhibiteur du métabolisme de RA endogène spécifique du CYP26A1, pourrait avoir un effet bénéfique sur la croissance et la différenciation cellulaire, en particulier celle des cellules épithéliales comme les kératinocytes.
D'une manière générale, on appelle ces inhibiteurs des agents bloqueurs du métabolisme de l'acide rétinoïque ou RAMBAs (pour "Retinoic acid Metabolism Blocking Agents"). Plus particulièrement, il conviendrait d'appeler de nouveaux composés inhibiteurs spécifiques du CYP26A1 des « RAMBA-CYP26A1 specific ». Ce type d'inhibiteur totalement spécifique n'a jamais été décrit à ce jour et constitue un enjeu pharmacologique et médical important.

Néanmoins, un certain nombre de composés RAMBA non spécifiques (retinoic acid metabolism blocking agents) sont déjà connus (Njar, V. C. O., ef a/., J. Bioorg. Med. Chem., 14, 4323 (2006)). Ils ont été décrits dans le traitement des désordres liés à un dysfonctionnement des cellules épithéliales (comme par exemple celles de l'épiderme) et des désordres liés à une concentration anormale en atRA dans certains tissus.

La première molécule RAMBA qui a été découverte comme étant efficace dans le traitement du psoriasis et l'ichtyose est le Liarozole (Kang et al. J Inv Dermatol, 104 (2) August 1996, 183-187). Le Liarozole a une faible spécificité enzymatique et peut donc inhiber d'autres voies médiées par les enzymes CYP, comme par exemple la biosynthèse de l'hormone surrénalienne. Ceci peut engendrer des effets secondaires indésirables attribués à un manque de spécificité de l'isoforme CYP. Par ailleurs, il est extrêmement complexe de gérer l'ensemble des interactions médicamenteuses pouvant survenir avec ce genre de composés compte tenu du large spectre de CYPs inhibés. Ces deux inconvénients (nombreux effets adverses potentiels et interactions médicamenteuses nombreuses) limitent grandement l'utilisation potentielle de ce type de composé, surtout chez des patients polymédicamentés.

Le Rambazole® (ou Talarozole) est un inhibiteur de seconde génération plus sélectif de CYP26 comparé à son activité sur les autres cytochromes comme le CYP3A4 et le CYP2C8 (Geria & Scheinfeld Curr. Opinion Invest. Drugs, 2008 9(11) : 12228-1237). Néanmoins, si l'on considère uniquement les CYP26, ce composé présente une sélectivité relativement faible pour le CYP26A1, avec une activité de l'ordre de 62 nM sur l'isoforme CYP26B1. Pour garantir une bonne spécificité de blocage d'un isoforme vis-à-vis d'un autre, il faut que le ratio des inhibitions soit à minima de 100.

Ainsi, il reste un besoin constant de mettre au point des composés utilisés dans des compositions cosmétiques et pharmaceutiques, et présentant une activité biologique améliorée comme inhibiteurs CYP26, en particulier CYP26A1.

### Résumé de l'invention

La Demanderesse a découvert des inhibiteurs CYP26 selon l'invention qui agissent comme des agents bloquants du métabolisme des acides rétinoïques (RAMBAs). Les composés possèdent une activité biologique améliorée comme inhibiteurs CYP26 ayant une grande sélectivité pour CYP26A1 par rapport au CYP26B1. Cette sélectivité accrue permet d'attendre une efficacité importante dans les organes où le CYP26A1 est bien exprimé comme la peau, tout en garantissant que les effets adverses pharmacologique « hors cible » médiés par le CYP26B1 seront très limités, voire inexistants. Le ratio bénéfice/risque attendu est donc nettement plus favorable pour ce genre d'inhibiteur pouvant garantir un niveau de sélectivité supérieur à 100 entre les 2 enzymes.

La présente invention concerne de nouveaux composés ou l'un de leurs sels répondant à la formule générale (I) suivante : 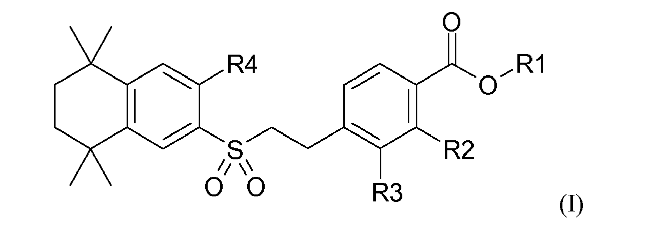 dans laquelle :
R1 représente un atome d'hydrogène ou un alkyle en C1-C3 ;
R2 représente un hydrogène, un alkyl en C1-C3, un fluor, ou un radical CF3 ;
R3 représente un hydrogène, un alkyl en C1-C3, un fluor, ou un radical CF3 ; et
R4 représente un hydrogène, un alkyl en C1-C3, ou un radical CF3.

De préférence, R1 est sélectionné dans le groupe constitué d'un hydrogène, un méthyle et un éthyle, de préférence un hydrogène ; R2 est sélectionné dans le groupe constitué d'un hydrogène, un méthyle, un fluor, ou un radical CF3 ; R3 est sélectionné dans le groupe constitué d'un hydrogène, un méthyle, un fluor, ou un radical CF3 ; et R4 est sélectionné dans le groupe constitué d'un hydrogène, un méthyle et un radical CF3.

Les composés préférés de formule (I) selon la présente invention sont choisi parmi :
- acide 4-[2-(5,5,8,8-tetramethyl-5,6,7,8-tetrahydro-naphthalene-2-sulfonyl)-ethyl]-benzoique ;
- acide 4-[2-(5,5,8,8-tetramethyl-5,6,7,8-tetrahydro-naphthalene-2-sulfonyl)-ethyl]-3-trifluoromethyl-benzoique ;
- acide 4-[2-(3,5,5,8,8-pentamethyl-5,6,7,8-tetrahydro-naphthalene-2-sulfonyl)-ethyl]-3-trifluoromethyl-benzoique ;
- 4-[2-(5,5,8,8-tetramethyl-5,6,7,8-tetrahydro-naphthalene-2-sulfonyl)-ethyl]-benzoate de méthyle ;
- 4-[2-(5,5,8,8-tetramethyl-5,6,7,8-tetrahydro-naphthalene-2-sulfonyl)-ethyl]-benzoate d'éthyle ;
- 4-[2-(5,5,8,8-tetramethyl-5,6,7,8-tetrahydro-naphthalene-2-sulfonyl)-ethyl]-3-trifluoromethyl-benzoate de méthyle ;
- 4-[2-(5,5,8,8-tetramethyl-5,6,7,8-tetrahydro-naphthalene-2-sulfonyl)-ethyl]-3-trifluoromethyl-benzoate d'éthyle;
- acide 2-méthyl-4-[2-(5,5,8,8-tetramethyl-5,6,7,8-tetrahydro-naphthalene-2-sulfonyl)-ethyl]-benzoique ;
- acide 2-fluoro-4-[2-(5,5,8,8-tetramethyl-5,6,7,8-tetrahydro-naphthalene-2-sulfonyl)-ethyl]-benzoique ;
- acide 4-[2-(5,5,8,8-tetramethyl-5,6,7,8-tetrahydro-naphthalene-2-sulfonyl)-ethyl]-2-trifluorométhyl-benzoique ;
- acide 3-methyl-4-[2-(5,5,8,8-tetramethyl-5,6,7,8-tetrahydro-naphtalene-2-sulfonyl)-ethyl]-benzoique ;
- acide 3-fluoro-4-[2-(5,5,8,8-tetramethyl-5,6,7,8-tetrahydro-naphtalene-2-sulfonyl)-ethyl]-benzoique ;
- acide 4-[2-(5,5,8,8-tetramethyl-3-trifluoromethyl-5,6,7,8-tetrahydro-naphtalene-2-sulfonyl)-ethyl]-benzoique ;
- acide 3-methyl-4-[2-(3,5,5,8,8-pentamethyl-5,6,7,8-tetrahydro-naphtalene-2-sulfonyl)-ethyl]-benzoique ;
- acide 2-methyl-4-[2-(3,5,5,8,8-pentamethyl-5,6,7,8-tetrahydro-naphtalene-2-sulfonyl)-ethyl]-benzoique ;
- acide 2,3-dimethyl-4-[2-(5,5,8,8-tetramethyl-5,6,7,8-tetrahydro-naphtalene-2-sulfonyl)-ethyl]-benzoique ;
- acide 2,3-difluoro-4-[2-(5,5,8,8-tetramethyl-5,6,7,8-tetrahydro-naphtalene-2-sulfonyl)-ethyl]-benzoique ; et
- 2-methyl-4-[2-(3,5,5,8,8-pentamethyl-5,6,7,8-tetrahydro-naphtalene-2-sulfonyl)-ethyl]-benzoate de méthyle.

Préférentiellement, les composés de formule (I) sont choisi parmi le groupe constituant en:
- acide 4-[2-(5,5,8,8-tetramethyl-5,6,7,8-tetrahydro-naphthalene-2-sulfonyl)-ethyl]-benzoique ;
- acide 4-[2-(5,5,8,8-tetramethyl-5,6,7,8-tetrahydro-naphthalene-2-sulfonyl)-ethyl]-3-trifluoromethyl-benzoique ; et
- acide 4-[2-(3,5,5,8,8-pentamethyl-5,6,7,8-tetrahydro-naphthalene-2-sulfonyl)-ethyl]-3-trifluoromethyl-benzoique ; et de manière encore plus préféré le composé de formule (I) est l'acide 4-[2-(5,5,8,8-tetramethyl-5,6,7,8-tetrahydro-naphthalene-2-sulfonyl)-ethyl]-benzoique.

La présente invention concerne également une composition comprenant un composé de formule (I) en association avec un solvant ou un support. De préférence, la composition est une composition pharmaceutique ou cosmétique et le solvant ou support est dermatologiquement acceptable.

Un composé ou la composition de l'invention peut être utilisé en tant que médicament, particulièrement pour prévenir ou traiter les pathologies suivantes :
1) les affections dermatologiques liées à un désordre de la kératinisation portant sur la différenciation et sur la prolifération cellulaire notamment pour traiter les acnés vulgaires, comédoniennes, rosacées, les acnés nodulokystiques, conglobata, les acnés séniles, les acnés secondaires telles que l'acné solaire, médicamenteuse ou professionnelle ;
2) les troubles de la kératinisation, notamment les ichtyoses, les états ichtyosiformes, l'ichtyose lamellaire, la maladie de Darrier, les kératodermies palmoplantaires, les leucoplasies, le pityriasis rubra pilaire et les états leucoplasiformes, le lichen cutané ou muqueux (buccal) ;
3) les affections dermatologiques avec une composante immuno-allergique inflammatoire, avec ou sans trouble de la prolifération cellulaire, et notamment toutes les formes de psoriasis, qu'il soit cutané, muqueux ou unguéal, et même le rhumatisme psoriasique, ou encore la dermatite atopique et les différentes formes d'eczéma;
4) les désordres cutanés dus à une exposition aux rayonnements U.V. pour réduire les pigmentations et les kératoses actiniques, ou toutes pathologies associées au vieillissement chronologique ou actinique, telle la xérose, les lentigines, le lentigo;
5) Toute condition liée à des proliférations dermiques ou épidermiques bénignes, qu'elles soient ou non d'origine virale telles que verrues vulgaires, les verrues planes, le molluscum contagiosum et l'épidermodysplasie verruciforme, les papillomatoses orales ou florides;
6) les désordres dermatologiques tels que les dermatoses immunes comme le lupus érythémateux, les maladies immunes bulleuses et les maladies du collagène, telle la sclérodermie ;
7) les stigmates de l'atrophie épidermique et/ou dermique induite par les corticostéroïdes locaux ou systémiques, ou toute autre forme d'atrophie cutanée,
8) les troubles de la cicatrisation, ou pour prévenir ou pour réparer les vergetures, ou encore pour favoriser la cicatrisation,
9) les affections d'origine fongique au niveau cutané tel que le tinea pedis et le tinea versicolor,
10) les désordres de la pigmentation, tels que l'hyperpigmentation, le mélasma, l'hypopigmentation ou le vitiligo ;
11) les états cancéreux ou précancéreux, cutanés ou muqueux comme les kératoses actiniques, la maladie de Bowen, les carcinomes in-situ, le kératoacanthome et les cancers cutanés comme le carcinome basocellulaire (BCC), le carcinome spinocellulaire (SCC) et les lymphomes cutanés tels que le lymphome T, la leucémie aiguë promyélocytaire, les neuroblastomes, les leucémies myéloïdes aiguëes, le cancer de la prostate, le cancer du sein.

De préférence, le composé ou la composition tel que défini ci-dessus est utilisé pour le traitement de maladies dermatologiques telles que l'acné, les ichtyoses, les états ichtyosiformes, l'hyperkératose palmoplantaire ou le psoriasis.

Le composé ou la composition de l'invention est également utilisée pour le traitement du vieillissement cutané. De préférence, la présente invention est toute particulièrement relative à l'utilisation pour le traitement de l'atrophie cutanée, des taches de pigmentations, des rides ou ridules, de la sécheresse cutanée, de la rugosité cutanée, de l'élastose solaire, et des télangiectasies.

La présente invention est également relative à l'utilisation cosmétique d'un composé ou d'une composition selon l'invention pour le traitement ou la prévention des taches de pigmentation, pour la diminution de la pigmentation de la peau, pour le traitement ou la prévention de l'atrophie cutanée, pour le traitement ou la prévention du vieillissement de la peau, notamment des rides et/ou ridules, pour le traitement ou la prévention des effets de tout agent physique ou chimique provoquant une accélération du vieillissement cutané, pour l'amélioration de l'élasticité et/ou du tonus de la peau et/ou pour le raffermissement de la peau, .

### Brève description des figures

Figure 1 : Schéma de synthèse générale des composés de l'invention. Dans un mode particulier, R1 est un méthyle.
Figure 2 : Photographie du stratum corneum chez un rat traité avec un véhicule.
Figure 3 : Photographie du stratum corneum chez un rat traité avec le composé 1 de l'invention.

### Description détaillée de l'invention

Les inventeurs ont découvert et identifié des nouveaux composés présentant un effet inhibiteur CYP26 et présentant également une grande sélectivité pour CYP26A1 par rapport au CYP26B1. Ces composés peuvent être utilisés dans des compositions pharmaceutiques et cosmétiques, seule ou en combinaison avec d'autres composés, en particulier un composé de la famille de la vitamine A, afin de promouvoir des effets bénéfiques sur la santé et l'esthétique.

La présente invention concerne donc de nouveaux composés ou l'un de leurs sels répondant à la formule générale (I) suivante : 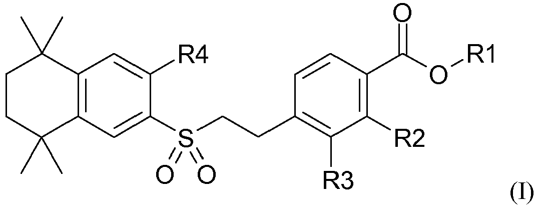 dans laquelle :
R1 représente un atome d'hydrogène ou un alkyle en C1-C3 ;
R2 représente un hydrogène, un alkyl en C1-C3, un fluor, ou un radical CF3 ;
R3 représente un hydrogène, un alkyl en C1-C3, un fluor, ou un radical CF3 ; et
R4 représente un hydrogène, un alkyl en C1-C3, ou un radical CF3.

Dans un premier mode de réalisation préférentiel, R1 est sélectionné dans le groupe constitué d'un hydrogène, un méthyle et un éthyle. En particulier, R1 est un hydrogène. Dans un deuxième mode de réalisation préférentiel, R2 est sélectionné dans le groupe constitué d'un hydrogène, un méthyle, un fluor, ou un radical CF3.
Dans un troisième mode de réalisation préférentiel, R3 est sélectionné dans le groupe constitué d'un hydrogène, un méthyle, un fluor, ou un radical CF3.
Dans un quatrième mode de réalisation préférentiel, R4 est sélectionné dans le groupe constitué d'un hydrogène, un méthyle et un radical CF3.

De préférence, R1 est sélectionné dans le groupe constitué d'un hydrogène, un méthyle et un éthyle ; R2 et R3, indépendamment l'un de l'autre, sont sélectionnés dans le groupe constitué d'un hydrogène, un méthyle, un fluor, ou un radical CF3 ; et R4 est sélectionné dans le groupe constitué d'un hydrogène, un méthyle et un radical CF3.
Dans un mode de réalisation particulier, R1 est sélectionné dans le groupe constitué d'un hydrogène et un alkyle en C1-C3, de préférence un méthyle et un éthyle ; et R2, R3 et R4 sont des hydrogènes.

A moins qu'il en soit indiqué autrement, les définitions suivantes s'appliquent à l'ensemble de la description et des revendications.

"Alkyle" désigne une chaîne hydrocarbonée saturée linéaire ou ramifiée dont le nombre d'atomes de carbone est précisé.
Les groupes alkyles préférés contiennent de 1 à 3 atomes de carbone dans la chaîne. Ils comprennent le méthyle, l'éthyle et le propyle, en particulier l'isopropyle.

A titre d'exemple de composés répondant à la formule générale (I), on peut citer les composés suivants sans que cette liste soit limitative :
- acide 4-[2-(5,5,8,8-tetramethyl-5,6,7,8-tetrahydro-naphthalene-2-sulfonyl)-ethyl]-benzoique;
- acide 4-[2-(5,5,8,8-tetramethyl-5,6,7,8-tetrahydro-naphthalene-2-sulfonyl)-ethyl]-3-trifluoromethyl-benzoique;
- acide 4-[2-(3,5,5,8,8-pentamethyl-5,6,7,8-tetrahydro-naphthalene-2-sulfonyl)-ethyl]-3-trifluoromethyl-benzoique;
- 4-[2-(5,5,8,8-tetramethyl-5,6,7,8-tetrahydro-naphthalene-2-sulfonyl)-ethyl]-benzoate de méthyle ;
- 4-[2-(5,5,8,8-tetramethyl-5,6,7,8-tetrahydro-naphthalene-2-sulfonyl)-ethyl]-benzoate d'éthyle ;
- 4-[2-(5,5,8,8-tetramethyl-5,6,7,8-tetrahydro-naphthalene-2-sulfonyl)-ethyl]-3-trifluoromethyl-benzoate de méthyle ;
- 4-[2-(5,5,8,8-tetramethyl-5,6,7,8-tetrahydro-naphthalene-2-sulfonyl)-ethyl]-3-trifluoromethyl-benzoate d'éthyle ;
- acide 2-méthyl-4-[2-(5,5,8,8-tetramethyl-5,6,7,8-tetrahydro-naphthalene-2-sulfonyl)-ethyl]-benzoique;
- acide 2-fluoro-4-[2-(5,5,8,8-tetramethyl-5,6,7,8-tetrahydro-naphthalene-2-sulfonyl)-ethyl]-benzoique;
- acide 4-[2-(5,5,8,8-tetramethyl-5,6,7,8-tetrahydro-naphthalene-2-sulfonyl)-ethyl]-2-trifluorométhyl-benzoique;
- acide 3-methyl-4-[2-(5,5,8,8-tetramethyl-5,6,7,8-tetrahydro-naphtalene-2-sulfonyl)-ethyl]-benzoique;
- acide 3-fluoro-4-[2-(5,5,8,8-tetramethyl-5,6,7,8-tetrahydro-naphtalene-2-sulfonyl)-ethyl]-benzoique;
- acide 4-[2-(5,5,8,8-tetramethyl-3-trifluoromethyl-5,6,7,8-tetrahydro-naphtalene-2-sulfonyl)-ethyl]-benzoique;
- acide 3-methyl-4-[2-(3,5,5,8,8-pentamethyl-5,6,7,8-tetrahydro-naphtalene-2-sulfonyl)-ethyl]-benzoique;
- acide 2-methyl-4-[2-(3,5,5,8,8-pentamethyl-5,6,7,8-tetrahydro-naphtalene-2-sulfonyl)-ethyl]-benzoique;
- acide 2,3-dimethyl-4-[2-(5,5,8,8-tetramethyl-5,6,7,8-tetrahydro-naphtalene-2-sulfonyl)-ethyl]-benzoique;
- acide 2,3-difluoro-4-[2-(5,5,8,8-tetramethyl-5,6,7,8-tetrahydro-naphtalene-2-sulfonyl)-ethyl]-benzoique; et
- 2-methyl-4-[2-(3,5,5,8,8-pentamethyl-5,6,7,8-tetrahydro-naphtalene-2-sulfonyl)-ethyl]-benzoate de méthyle.

De préférence, le composé de formule générale (I) est choisi parmi le groupe consistant en
- acide 4-[2-(5,5,8,8-tetramethyl-5,6,7,8-tetrahydro-naphthalene-2-sulfonyl)-ethyl]-benzoique;
- acide 4-[2-(5,5,8,8-tetramethyl-5,6,7,8-tetrahydro-naphthalene-2-sulfonyl)-ethyl]-3-trifluoromethyl-benzoique; et
- acide 4-[2-(3,5,5,8,8-pentamethyl-5,6,7,8-tetrahydro-naphthalene-2-sulfonyl)-ethyl]-3-trifluoromethyl-benzoique.

Un composé préféré selon l'invention est l'acide 4-[2-(5,5,8,8-tetramethyl-5,6,7,8-tetrahydro-naphthalene-2-sulfonyl)-ethyl]-benzoique.

La présente invention concerne une composition comprenant un composé répondant à la formule générale (I) ou d'un de ses sels tel que défini ci-dessus en association avec un solvant ou un support.
En particulier, la composition est une composition cosmétique et le solvant ou support est dermatologiquement acceptable. Ainsi, la composition comprend une quantité cosmétiquement efficace du composé, c'est-à-dire suffisante pour permettre un effet cosmétique.

La présente invention concerne également une composition pharmaceutique comprenant une quantité efficace d'un composé répondant à la formule générale (I) ou d'un sel pharmaceutiquement acceptable du dit composé tel que décrit ci-dessus en association avec un solvant ou un support pharmaceutiquement acceptable, de préférence dermatologiquement acceptable. Ainsi, la composition comprend une quantité thérapeutiquement efficace du composé, c'est-à-dire suffisante pour permettre un effet thérapeutique ou pharmaceutique.

Les composés de l'invention sous forme de sels, en particulier sous forme de sels dermatologiquement, pharmaceutiquement ou cosmétiquement acceptables incluent notamment les sels d'addition acides, les sels d'addition basiques, les sels métalliques, et les sels d'ammonium ou d'ammonium alkylé, en particulier ceux pharmaceutiquement ou cosmétiquement acceptables. Les sels d'addition acides incluent les sels inorganiques et les sels organiques. Des exemples représentatifs d'acides inorganiques adaptés comprennent les acides chlorhydrique, bromhydrique, iodique, phosphorique, . Des exemples représentatifs d'acides organiques adaptés comprennent les acides acétique, formique, propionique, benzoïque, maléique, fumarique, succinique, tartrique, citrique, trichloroacétique, trifluoroacétique. D'autres exemples de sels d'addition acides organiques ou inorganiques sont fournis dans J. Pharm Sci., 1977, 66, 2 et dans « Handbook of Pharmaceutical Salts : Properties, Selection and Use édité par P. Heinrich Stahl et Camille G. Wermuth, 2002. Des exemples de sels métalliques incluent les sels de lithium, sodium, potassium, ou magnésium, .Des exemples de sels d'ammonium et d'ammonium alkylés incluent les sels d'ammonium, de méthyl-ammonium, de diméthyl-ammonium, de diméthyl-ammonium, de triméthyl-ammonium, d'éthyl-ammonium, d'hydroxyéthyl-ammonium, de diéthyl-ammonium, de butyl-ammonium, tétraméthyl-ammonium, .Des exemples de bases organiques incluent la lysine, l'arginine, la guanidine, la diéthanolamineoline

La présente invention concerne un composé ou une composition pharmaceutique tel que décrit ci-dessus pour son utilisation en tant que médicament. Elle concerne également l'utilisation d'un composé ou d'une composition selon la présente demande pour la préparation d'un médicament. Elle concerne enfin des méthodes de traitement comprenant l'administration d'une quantité efficace d'un composé ou d'une composition selon la présente demande.

La présente invention concerne donc les composés selon l'invention, ou une composition comprenant au moins un composé selon l'invention, ou l'un de ses sels pharmaceutiquement acceptables, pour une utilisation pour traiter les pathologies suivantes :
1) les affections dermatologiques liées à un désordre de la kératinisation portant sur la différenciation et sur la prolifération cellulaire notamment pour traiter les acnés vulgaires, comédoniennes, rosacées, les acnés nodulokystiques, conglobata, les acnés séniles, les acnés secondaires telles que l'acné solaire, médicamenteuse ou professionnelle ;
2) les troubles de la kératinisation, notamment les ichtyoses, les états ichtyosiformes, l'ichtyose lamellaire, la maladie de Darrier, les kératodermies palmoplantaires, les leucoplasies, le pityriasis rubra pilaire et les états leucoplasiformes, le lichen cutané ou muqueux (buccal) ;
3) les affections dermatologiques avec une composante immuno-allergique inflammatoire, avec ou sans trouble de la prolifération cellulaire, et notamment toutes les formes de psoriasis, qu'il soit cutané, muqueux ou unguéal, et même le rhumatisme psoriasique, ou encore la dermatite atopique et les différentes formes d'eczéma;
4) les désordres cutanés dus à une exposition aux rayonnements U.V. pour réduire les pigmentations et les kératoses actiniques, ou toutes pathologies associées au vieillissement chronologique ou actinique, telle la xérose, les lentigines, le lentigo ;
5) Toute condition liée à des proliférations dermiques ou épidermiques bénignes, qu'elles soient ou non d'origine virale telles que verrues vulgaires, les verrues planes, le molluscum contagiosum et l'épidermodysplasie verruciforme, les papillomatoses orales ou florides;
6) les désordres dermatologiques tels que les dermatoses immunes comme le lupus érythémateux, les maladies immunes bulleuses et les maladies du collagène, telle la sclérodermie;
7) les stigmates de l'atrophie épidermique et/ou dermique induite par les corticostéroïdes locaux ou systémiques, ou toute autre forme d'atrophie cutanée ;
8) les troubles de la cicatrisation, ou pour prévenir ou pour réparer les vergetures, ou encore pour favoriser la cicatrisation ;
9) les affections d'origine fongique au niveau cutané tel que le tinea pedis et le tinea versicolor ;
10) les désordres de la pigmentation, tels que l'hyperpigmentation, le mélasma, l'hypopigmentation ou le vitiligo;
11) les états cancéreux ou précancéreux, cutanés ou muqueux comme les kératoses actiniques, la maladie de Bowen, les carcinomes in-situ, le kératoacanthome et les cancers cutanés comme le carcinome basocellulaire (BCC), le carcinome spinocellulaire (SCC) et les lymphomes cutanés tels que le lymphome T, la leucémie aiguë promyélocytaire, les neuroblastomes, les leucémies myéloïdes aiguëes, le cancer de la prostate, le cancer du sein.

Dans un mode de réalisation préféré selon l'invention, le composé ou la composition pharmaceutique est utilisé dans le traitement de maladies dermatologiques telles que l'acné, les ichtyoses, les états ichtyosiformes, l'hyperkératose palmoplantaire ou le psoriasis.

La présente invention concerne également l'utilisation d'un composé ou d'une composition selon l'invention pour le traitement du vieillissement cutané.

Par vieillissement cutané, on entend le vieillissement chronologique et/ou actinique, où l'épiderme subit de nombreuses modifications et dégradations qui se traduisent, avec l'âge, par une altération du microrelief, une apparition de rides et ridules, une altération des propriétés mécaniques de la peau avec diminution de la fermeté et de l'élasticité et une perte d'éclat du teint. Au niveau tissulaire, le vieillissement se manifeste par une désorganisation de l'architecture épidermique, de la jonction dermo-épidermique et du derme, ainsi que des systèmes d'irrigation sanguine et d'innervation, un ralentissement de différents métabolismes tels que ceux mis enjeu dans l'équilibre de la fonction barrière. Au niveau cellulaire, le vieillissement se traduit par une altération de la physiologie ou du métabolisme des principaux types cellulaires du derme (fibroblastes) et de l'épiderme (kératinocytes). Le vieillissement chronologique ou le vieillissement lié à une exposition solaire (photovieillissement) ou à un agent physique ou chimique, peut s'accompagner de taches de pigmentation, d'atrophie cutanée, des rides ou ridules, de la sécheresse cutanée, de la rugosité cutanée, d'élastose solaire mais également de télangiectasies qui sont de petits vaisseaux dilatés à la surface de la peau. Plus généralement et de manière non exhaustive, ce vieillissement lié à l'âge ou à une exposition solaire ou à un agent physique ou chimique est responsable d'une perte d'élasticité, souplesse et de tonicité de la peau par modification des protéines de soutien du derme comme les collagènes, des modifications de paramètres physiologiques et biophysiques comme l'hydratation de la peau ou ses propriétés de barrière. Il résulte également de ce vieillissement cutané une modification générale de l'aspect de la peau, de sa couleur, finesse, grain et douceur. Ces différentes propriétés de la peau peuvent être modifiées de manière bénéfique par un traitement avec les composés et les compositions de l'invention.

La présente invention est donc particulièrement relative à l'utilisation cosmétique d'un composé ou d'une composition selon l'invention pour maintenir et/ou stimuler l'hydratation de la peau, pour le traitement des peaux sèches ou pour le traitement ou la prévention du vieillissement de la peau ou des phanères et/ou du photo-vieillissement, en particulier pour le traitement ou la prévention des taches de pigmentation, des rides, notamment les rides profondes et/ou les ridules, pour diminuer la ptôse, ou améliorer la qualité et/ou la transparence de la peau, pour l'amélioration ou la restauration de la souplesse, de l'élasticité et/ou du tonus de la peau, pour le raffermissement de la peau.

La quantité de composés selon l'invention utilisés dans les compositions de l'invention dépend de l'effet cosmétique ou thérapeutique et peut donc varier. Une gamme de concentration du composé de l'invention peut être comprise entre environ 0,001 g/L ou g/Kg et environ 1000 g/L ou g/kg de composition, et de préférence comprise entre environ 0,1 g/L ou g/kg et environ 10 g/L ou g/kg de composition semble adaptée pour les compositions de l'invention.

Les compositions selon l'invention comprennent une dose allant de 0,000001 % à 10 %, préférentiellement de 0,001 à 5 %, et encore plus préférentiellement de 0,1 à 3% en poids total d'un composé de l'invention par rapport au poids total de la composition.

La composition selon l'invention peut être formulée pour être adaptée à une administration par voie orale, par voie topique sur la peau, ou par injection intraépidermique et/ou intradermique et/ou transdermique et/ou sous-cutanée et/ou micro-injection.

La composition selon la présente invention pour une administration topique peut être sous la forme de solutions aqueuses, hydroalcooliques, d'émulsions (huile dans l'eau (H/E), eau dans l'huile (E/H), ou multiple (par exemple triple H/E/H ou E/H/E), de nanoémulsions, de gels aqueux ou de dispersions de phase grasse dans une phase aqueuse. Elle peut être formulée sous forme d'une crème plus ou moins fluide, d'un gel, d'un hydrogel, d'un produit filmogène (notamment à base d'acide hyaluronique de haut poids moléculaire qui a la propriété de capturer l'eau et de former un gel à l'étalement), d'une lotion, de masque, d'un lait, d'une huile, d'un onguent, d'une cire, d'une mousse, d'une pâte, d'un sérum, d'une pommade, d'un aérosol, d'un stick, d'un savon ou d'un shampoing.

La composition adaptée à une administration topique peut être mise en oeuvre en combinaison avec des éléments mécaniques tels que des dispositifs à palper-rouler ayant une action mécanique et massante faisant pénétrer le produit et activant la circulation du produit ou des systèmes émetteurs d'ondes (lumière, basses fréquences, fréquences infrarouges, ) qui permettent d'activer la réponse de la peau ou d'augmenter directement l'effet du produit.

La composition selon la présente invention pour une administration orale peut être sous la forme de cachet, pilule, gélule, sirop ou comprimé.

La composition selon la présente invention pour une administration injectable est de préférence formulée sous une forme stérile. Elle peut aussi se présenter sous forme de poudre, le solvant étant ajouté extemporanément au moment de l'utilisation.

Les composants de la composition ainsi que leurs proportions peuvent être aisément choisis par l'homme du métier sur la base de ses connaissances générales.
La composition peut être également comprise dans un dispositif adapté au mode d'administration considéré.

De tels dispositifs peuvent être adaptés à une administration topique. On peut notamment citer les patchs, les pansements occlusifs, les mini-chambres d'occlusion, etc... Par patch, on entend tout type de patch connu de l'homme du métier, et notamment ceux qui génèrent un léger courant au niveau de la peau lorsqu'il est collé sur celle-ci permettant de favoriser la pénétration du produit dans celle-ci. Par mini-chambres d'occlusion, on entend un dispositif du type petite poche qui se colle à la peau, remplie de la composition et qui permet de maintenir une quantité importante de cette composition au contact de la peau pour augmenter la quantité de produit qui va diffuser dans celle-ci.

D'autres dispositifs seront adaptés à une injection, en particulier adaptée à une injection intraépidermique et/ou intradermique et/ou transdermique et/ou sous-cutanée et/ou micro-injection. De tels dispositifs peuvent comprendre une aiguille, des micro-aiguilles ou un dispositif d'injection sans aiguille. De tels dispositifs sont bien connus en mésothérapie. L'invention concerne des kits comprenant la composition selon la présente invention et une seringue ou un dispositif injecteur.
Les composés de formule générale (I) de la présente invention peuvent être préparés suivant la voie de synthèse telle que décrite ci-dessous ou telles qu'elles ressortent des différents exemples de préparation donnés ci-après de façon non limitative.

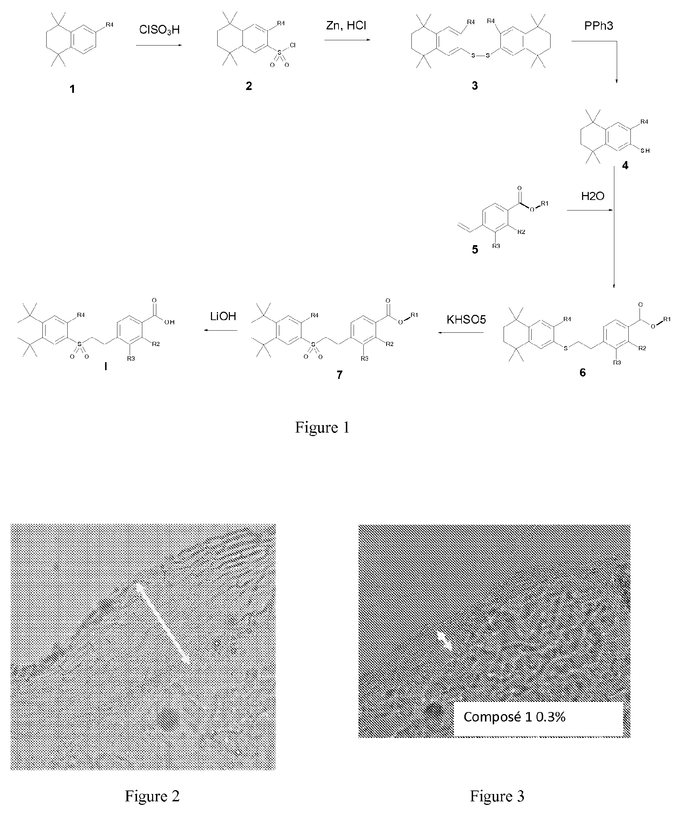

La voie de synthèse générale pour la préparation des composés de formule (I) est illustrée sur le **schéma 1** (Figure 1). La synthèse des dérivés arylthiols **4** peut être réalisée par réaction des arènes **1** avec de l'acide chlorosulfonique pour fournir les arènes chlorosulfoniques **2** correspondants qui sont ensuite réduits par un réducteur comme la poudre de zinc en présence d'acide chlorhydrique par exemple pour conduire aux disulfures de formule générale **3.** Ces disulfures sont ensuite réduits par exemple par réaction avec la triphenylphosphine dans un milieu dioxanne/eau pour conduire aux thiols de formule générale **4.**

Les dérivés de formule générale **4** peuvent s'additionner sur les dérivé vinyliques de formule générale **5** (commerciaux ou synthétisé par les méthodes connus de l'homme de l'art) dans un milieu aqueux, par exemple d'eau, (Synlett, 2007, 925-928) pour conduire au dérivés de formule générale **6.**

Les sulfures de la formule générale **6** peuvent ensuite être oxydés en leur sulfone correspondante de formule générale **7** par action d'un oxydant comme l'Hydrogénopersulfate de potassium (oxone®) par exemple. Les composés de formule générale I objet de la présente invention sont obtenus par saponification à l'aide d'une base comme l'hydroxyde de lithium par exemple des ester de formule générale **7** dans un solvant comme le THF, l'éthanol ou l'eau ou encore dans un mélange de ces solvants.

A titre d'illustration, les composés suivants répondant à la formule générale (I) de la présente invention ont pu être préparés en suivant l'un des schémas présentés ci-dessus.

### EXEMPLES

### Exemple 1 : acide 4-[2-(5,5,8,8-tetramethyl-5,6,7,8-tetrahydro-naphthalene-2-sulfonyl)-ethyl]-benzoique

### 5,5,8,8-Tetramethyl-5,6,7,8-tetrahydro-naphthalene-2-disulfide

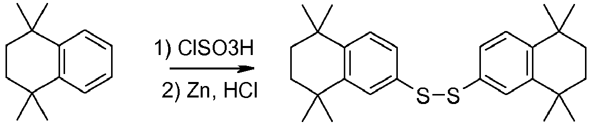

100 g (0.532 mol) de 1,1,4,4-Tetramethyl-1,2,3,4-tetrahydro-naphthalene sont ajoutés gouttes à gouttes sur 166.5 mL d'acide chlorosulfonique à 0°C, le milieu réactionnel est agité 3h à TA. Le mélange est versé sur de la glace et extrait avec de l'acétate d'éthyle puis le solvant est évaporé sous vide. 143 g de solide brun sont obtenus. Ce solide est repris par 660 mL d'éthanol, refroidi à 0°C puis 156.6 g de poudre de zinc sont ajoutés ainsi que 660 mL d'acide chlorhydrique 37%. Le mélange réactionnel est agité une nuit à TA puis filtré sur célite. Le solide plus la celite sont repris avec de l'acétate d'éthyle puis la phase acétate d'éthyle est lavée avec NaHCO3. Le solvant est évaporé pour donner 110 g d'un solide brun. Ce solide est cristallisé dans un mélange éther éthylique/éthanol pour fournir 64 g de 5,5,8,8-Tetramethyl-5,6,7,8-tetrahydro-naphthalene-2-disulfide sous forme de solide beige. Rdt = 27%.

### 5,5,8,8-Tetramethyl-5,6,7,8-tetrahydro-naphtalene-2-thiol

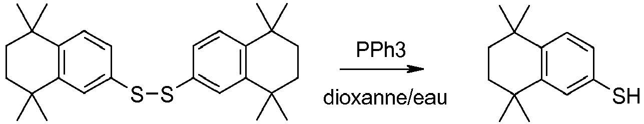

27 g (62.7 mmol) de disulfure 5,5,8,8-Tetramethyl-5,6,7,8-tetrahydro-naphthalene-2-disulfide précédent sont mis en suspension dans240 mL un mélange dioxanne/eau 9/1 puis 16.3 g (63 mmol) de triphenylphosphine sont ajoutés puis le mélange est porté 6 heures à reflux puis concentré à sec. Le résidu est filtré sur silice (DCM/hept 8/2) pour fournir 28g de 5,5,8,8-Tetramethyl-5,6,7,8-tetrahydro-naphthalene-2-thiol sous forme de solide jaune. Rdt = 100%.

### 4-[2-(5,5,8,8-Tetramethyl-5,6,7,8-tetrahydro-naphthalen-2-ylsulfanyl)-ethyl]-benzoate de méthyle

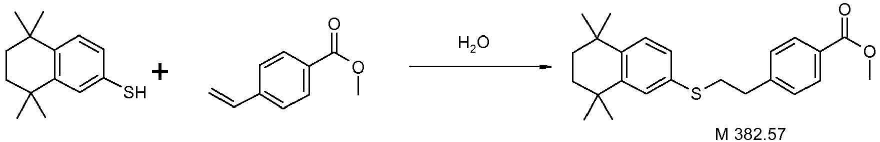

Sous azote, un mélange de 4-Vinyl-benzoate de méthyle (1.6g, 9.87mmol) et de 5,5,8,8-Tetramethyl-5,6,7,8-tetrahydronaphthalene-2-thiol (2.39 g, 10.85 mmol) en suspension dans 15 ml d'eau dégazée est agité à température ambiante pendant 24 heures. Le milieu réactionnel est repris avec de l'acétate d'éthyle et de l'eau. Après extraction, la phase organique est lavée avec une solution de soude 1N puis une solution de NaCl saturée, séchée sur MgSO₄ puis concentrée. Le composé obtenu est chromatographié sur une cartouche de gel de silice (Heptane/Acétate d'éthyle 97/3). On recueille m = 2.74 g de 4-[2-(5,5,8,8-Tetramethyl-5,6,7,8-tetrahydro-naphthalen-2-ylsulfanyl)-ethyl]-benzoate de méthyle sous forme de solide blanc Rdt = 74%

**RMN ¹H /DMSO, 400 MHz :** 1.21 (s, 6H) ; 1.22(s, 6H) ; 1.62 (s, 4H) ; 2.93 (t, j=7.6Hz, 2H) ; 3.24 (t, j=7.6Hz, 2H) ; 3.84 (s, 3H) ; 7.10 (dd, j=8.3Hz-j=2.0Hz, 1H) ; 7.22 (d, j=2.0Hz, 1H) ; 7.26 (d, j=8.3Hz, 1H) ; 7.39 (d, j=8.3Hz, 2H) ; 7.88 (d, j=8.2Hz, 2H).

### 4-[2-(5,5,8,8-Tetramethyl-5,6,7,8-tetrahydro-naphthalene-2-sulfonyl)-ethyl]-benzoate de méthyle (Composé 4)

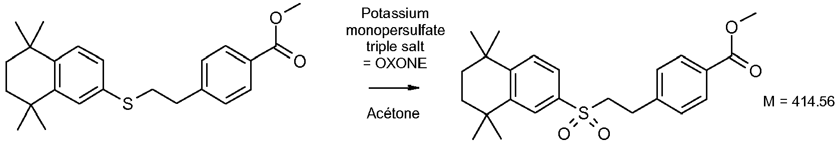

Au 4-[2-(5,5,8,8-Tetramethyl-5,6,7,8-tetrahydro-naphthalen-2-ylsulfanyl)-ethyl]-benzoate de méthyle (3.47 g, 9.07 mmol) en solution dans 136 ml d'acétone est ajouté du sel de Potassium monopersulfate triple (Oxone®) (16.73 g, 27.21 mmol). Le mélange réactionnel est agité la nuit à température ambiante. Le milieu est filtré et concentré pour donner 3.54 g de 4-[2-(5,5,8,8-Tetramethyl-5,6,7,8-tetrahydro-naphthalene-2-sulfonyl)-ethyl]-benzoate de méthyle sous forme d'un solide beige. Rdt =94%

**RMN ¹H/DMSO, 400 MHz :** 1.24 (s, 6H) ; 1.25 (s, 6H) ; 1.64 (s, 4H) ; 3.01 (t, j=7.8Hz, 2H) ; 3.74 (t, j=7.5Hz, 2H) ; 3.81 (s, 3H) ; 7.30 (d, j=8.3Hz, 2H) ; 7.52 (d, j=8.4Hz, 1H) ; 7.57 (dd, j=8.4Hz-j=1.90Hz, 1H) ; 7.74-7.77 (m, 3H).

### Acide 4-[2-(5,5,8,8-tetramethyl-5,6,7,8-tetrahydro-naphthalene-2-sulfonyl)-ethyl]-benzoique (Composé 1)

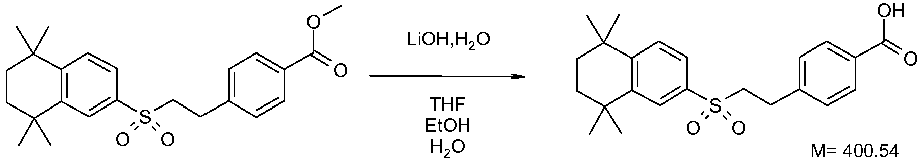

On place le 4-[2-(5,5,8,8-Tetramethyl-5,6,7,8-tetrahydro-naphthalene-2-sulfonyl)-ethyl]-benzoate de méthyle (3.54, 8.54 mmol) en solution dans 256 ml de tétrahydrofuranne, 25.6 ml d'éthanol et 12.8 ml d'eau dans un ballon. On ajoute 3.54 g de lithine monohydrate. Le tout est agité 4 heures à reflux. L'ambiant le milieu réactionnel est concentré de moitié repris avec de l'acétate d'éthyle et de l'eau puis amené à pH6 avec HCl 2N. Après extraction, la phase organique est lavée avec une solution de NaCl saturée, séchée sur MgSO₄, concentrée et lavé à l'éther. On obtient 3.g d'acide 4-[2-(5,5,8,8-Tetramethyl-5,6,7,8-tetrahydro-naphthalene-2-sulfonyl)-ethyl]-benzoique sous forme d'un solide blanc. Rdt = 88%, Tf 203°C.

**RMN ¹H /DMSO, 400 MHz :** 1.24 (s, 6H) ; 1.25(s, 6H) ; 1.64 (s, 4H) ; 2.99 (t, j=7.9Hz, 2H) ; 3.72 (t, j=7.5Hz, 2H) ; 7.26 (d, j=8.1Hz, 2H) ; 7.52-7.60 (m, 2H) ; 7.74-7.76 (m, 3H) ; 12.85 (s, 1H).
**RMN ¹³C /DMSO, 400 MHz :** 28.12(CH₂) ; 31.18 (CH₃) ; 31.26 (CH₃) ; 33.99 (CH₂) ; 34.06 (CH₂) ; 34.23 (C) ; 34.38 (C) ; 54.45 (CH₂) ; 124.52 (CH) ; 125.65 (CH) ; 127.67 (2CH) ; 128.57 (CH) ; 128.93 (C) ; 129.23 (2CH) ; 136.33 (C) ; 142.88 (C) ; 145.67 (C) ; 150.59 (C) ; 167.09 (C) ;

### Exemple 2: acide 4-[2-(5,5,8,8-tetramethyl-5,6,7,8-tetrahydro-naphthalene-2-sulfonyl)-ethyl]-3-trifluoromethyl-benzoique

De manière analogue à l'exemple 1 mais en utilisant l'acide 3-Trifluoromethyl-4-vinyl-benzoique on obtient l'acide 4-[2-(5,5,8,8-Tetramethyl-5,6,7,8-tetrahydro-naphthalene-2-sulfonyl)-ethyl]-3-trifluoromethyl-benzoique.

**RMN ¹H /CDCl₃, 400 MHz :** 1.24 (s, 6H) ; 1.25(s, 6H) ; 1.66 (s, 4H) ; 3.17-3.21 (m, 2H) ; 3.23-3.28 (m, 2H) ; 7.39 (d, j=8.1Hz, 1H) ; 7.44 (d, j=8.4Hz, 1H) ; 7.57 (dd, j=8.3Hz-j=2.0Hz, 1H) ; 7.78 (d, j=2.0Hz, 1H) ; 8.12 (dd, j=8.0Hz-j=3.6Hz, 1H) ; 8.26 (d, j=1.2Hz, 1H).
**RMN ¹³C /CDCl₃, 400 MHz :** 26.37 (CH₂) ; 29.71 (C) ; 31.60 (CH₃) ; 31.67 (CH₃) ; 34.53 (CH₂) ; 34.607 (CH₂) ; 34.71 (C) ; 34.86 (C) ; 56.76 (CH₂) ; 122.19 (C) ; 124.84 (CH) ; 124.91 (C) ; 126.60 (CH₂) ; 127.98 (CH) ; 128.27 (CH) ; 128.33 (CH) ; 128.39 (C) ; 128.57 (CH) ; 129.31 (CF₃) ; 131.83 (CH) ; 133.69 (C) ; 135.21 (C) ; 142.53 (C) ; 146.84 (C) ; 151.99 (C) ; 169.55 (C) ;

### Exemple 3 : acide 4-[2-(3,5,5,8,8-pentamethyl-5,6,7,8-tetrahydro-naphthalene-2-sulfonyl)-ethyl]-3-trifluoromethyl-benzoique

De manière analogue à l'exemple 1 mais en utilisant le 1,1,4,4,6-pentamethyl-1,2,3,4-tetrahydro-naphthalene et l'acide 3-trifluoromethyl-4-vinyl-benzoique on obtient l'acide 4-[2-(3,5,5,8,8-pentamethyl-5,6,7,8-tetrahydro-naphthalene-2-sulfonyl)-ethyl]-3-trifluoromethyl-benzoique.

**RMN ¹H /CDCl₃, 400 MHz :** 1.22 (s, 6H) ; 1.23(s, 6H) ; 1.63 (s, 4H) ; 2.53 (s, 3H) ; 3.17-3.22 (m, 2H) ; 3.25-3.30 (m, 2H) ; 7.18 (d, j=9.7Hz, 1H) ; 7.40 (d, j=7.4Hz, 1H) ; 7.87 (s, 1H) ; 8.12 (dd, j=8.0Hz-j=1.4Hz, 1H) ; 8.27 (d, j=1.2Hz, 1H).
**RMN ¹³C /CDCl₃, 400 MHz :** 19.94 (CH₃) ; 26.07 (CH₂) ; 29.71 (CH₂) ; 31.50 (CH₃) ; 31.61 (CH₃) ; 34.25 (C) ; 34.53 (C) ; 34.64 (CH₂) ; 34.67 (CH₂) ; 55.81 (CH₂) ; 128.28 (CH) ; 128.34 (CH₂) ; 128.96 (CH) ; 129.35 (CF₃) ; 131.19 (CH) ; 131.88 (CH) ; 133.43 (C) ; 133.68 (CH) ; 134.14 (C) ; 142.73 (C) ; 144.00 (C) ; 15180 (C) ; 169.39 (C) ;

### Exemple 4: Mesure de l'activité inhibitrice sur les isoformes de l'enzyme CYP26

Un test cellulaire utilisant des cellules HeLa surexprimant soit l'enzyme CYP26A1 ou CYP26B1 humaine, semblable à celui décrit dans (White et al., Proc Natl Acad Sci USA. 2000 Jun 6; 97(12): 6403-8) a été utilisé. Brièvement, les cellules ont été étalées dans des plaques multi-puits et traitées avec les composés selon l'invention ou les contrôles positifs tels que le liarozole ou le rambazole et par la suite exposées à de l'acide rétinoique radiomarqué (3H-RA). Après le temps d'incubation de 3h, la réaction a été arrêtée et une phase d'extraction a été réalisée pour séparer les couches aqueuses et organiques. La quantité de radioactivité présente dans la couche aqueuse a ensuite été mesurée comme un indicateur de la quantité de RA qui a été métabolisé.

Une première expérience en utilisant 3 différentes concentrations de composés selon l'invention (0,1, 1 et 10 µM) a été effectuée pour évaluer l'activité inhibitrice des composés. Un second essai a ensuite été réalisé avec une large gamme de concentrations (0,01, 0,1, 0,5, 1, 5 et 10 µM). Les résultats obtenus au cours de ces deux essais sont similaires.

Le tableau suivant répertorie la moyenne des AC50 obtenues sur ces deux expériences.

| **Moyenne AC50 (µM)** | **Liarozole** | **Rambazole** | **Exemple 1 selon l'invention** |
|---|---|---|---|
| **CYP26A1** | 4.00 | 0.004 | 0.19 |
| **CYP26B1** | 0.32 | 0.064 | 50.33 |
| **CYP26B1/CYP26A1** | 0.08 | 16.82 | 261.50 |
| **Spécificité CYP26A1/CYP26B1** | - | + | +++ |

Le rambazole est actif sur l'inhibition de l'activité des deux isoformes de l'enzyme.
Quant au Liarozole, celui-ci est plus actif sur_CYP26B1 que sur CYP26A1.
Les composé selon l'invention présente un ratio de sélectivité CYP26B1/CYP26A1 supérieur à 260, avec une activité très forte sur l'isoforme CYP26A1 de l'ordre de 0.1µM et une activité sur l'isoforme CYP26B1 extrêmement faible, voire négligeable, de l'ordre de 50µM.

Ces résultats démontrent clairement que les composés selon l'invention agissent comme un RAMBA à action directe sur CYP26. Les composés selon l'invention démontrent une grande sélectivité pour l'isoforme CYP26A1 comparée à l'isoforme CYP26B1.

### Exemple 5 : Mesure de l'épaisseur du stratum corneum chez le rat Fuzzy

Des rats males Fuzzy sont séparés en quatre lots comportant chacun cinq rats. Un traitement topique est réalisé sur chaque lot en administrant respectivement, un véhicule jouant le rôle de témoin, une dose de 0,000001% de rétinol, une dose de 0,1% de rétinol et une dose 0,3 % d'un composé selon l'invention correspondant à l'exemple 1.

24 jours après le traitement, les rats sont euthanasiés et une section de 10µm de peau est prélevée pour réaliser une biopsie. Une solution de NaOH 1M est ajoutée sur chaque section pour assouplir le stratum corneum. Les photos sous microscope sont réalisées dans un laps de temps de 2 minutes après l'ajout de la solution de NaOH 1M.

Le tableau ci-dessous présente les résultats sur l'épaisseur du stratum corneum pour les différents lots.

| | Epaisseur du stratum corneum (µm) | Epaisseur du stratum corneum par rapport au véhicule |
|---|---|---|
| Véhicule | 42,59 | 1 |
| Composé 1, 0,3 % | 24,07 | 0,57 |
| Rétinol 0,000001 % | 41,60 | 0,98 |
| Rétinol 0,1 % | 26,24 | 0,62 |

Une épaisseur de 41,60 µm du stratum corneum est mesurée chez les rats traités avec 0,000001 % de rétinol. Cette épaisseur est sensiblement équivalente à celle du lot témoin ayant reçu un simple véhicule (42,59 µm).

Une diminution de l'épaisseur du stratum corneum chez les rats traités avec 0,1% de rétinol (0.62) et chez les rats traités avec le composé 1 de l'invention (0,57) est observée.

La diminution de l'épaisseur du stratum corneum est même légèrement supérieure avec le composé 1, comparé avec 0,1 % de rétinol servant de référence.

Les résultats sont également illustrés par les figures 2 et 3 qui représentent des photographies de la couche cornée chez les rats ayant reçus le véhicule et chez les rats traités avec 0,3 % du composé 1 de l'invention. Les photographies montrent clairement la diminution de l'épaisseur du stratum corneum pour les rats traités avec 0,3 % du composé 1 de l'invention.

Ces résultats démontrent clairement que les composés selon l'invention permettent ainsi de diminuer l'épaisseur du stratum corneum et peuvent donc être utilisés dans des traitements, notamment en affinant la peau, plus particulièrement contre le vieillissement cutané, l'acné par la diminution de l'hyperplasie des keratinocytes au niveau du canal pylosebacé (origine des microcomédons) et de la formations des lésions d'acné, mais également l'ichtyose par l'élimination de l'ensemble des zones hyperplasiques et hyperkeratinisées.

## Revendications

1. Composés ou l'un de leurs sels de formule générale (I) suivante : 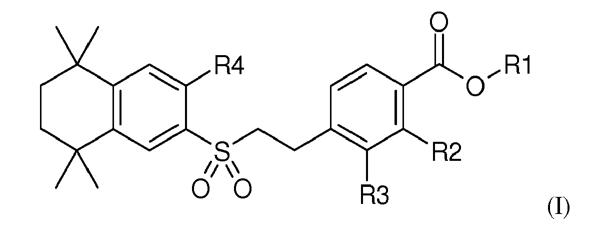 dans laquelle :
R1 représente un atome d'hydrogène ou un alkyle en C1-C3 ;
R2 représente un hydrogène, un alkyl en C1-C3, un fluor, ou un radical CF3 ;
R3 représente un hydrogène, un alkyl en C1-C3, un fluor, ou un radical CF3 ;
R4 représente un hydrogène, un alkyl en C1-C3, ou un radical CF3.

2. Composé selon la revendication 1, **caractérisé en ce que** :
R1 est sélectionné dans le groupe constitué d'un hydrogène, un méthyle et un éthyle;
R2 est sélectionné dans le groupe constitué d'un hydrogène, un méthyle, un fluor, ou un radical CF3 ;
R3 est sélectionné dans le groupe constitué d'un hydrogène, un méthyle, un fluor, ou un radical CF3 ; et
R4 est sélectionné dans le groupe constitué d'un hydrogène, un méthyle et un radical CF3.

3. Composé selon la revendication 1 ou 2, **caractérisé en ce que** R1 représente un atome d'hydrogène.

4. Composé selon l'une quelconque des revendications 1 à 3, **caractérisé en ce qu'**il est choisi dans le groupe constitué par :
- acide 4-[2-(5,5,8,8-tetramethyl-5,6,7,8-tetrahydro-naphthalene-2-sulfonyl)-ethyl]-benzoique ;
- acide 4-[2-(5,5,8,8-tetramethyl-5,6,7,8-tetrahydro-naphthalene-2-sulfonyl)-ethyl]-3-trifluoromethyl-benzoique ;
- acide 4-[2-(3,5,5,8,8-pentamethyl-5,6,7,8-tetrahydro-naphthalene-2-sulfonyl)-ethyl]-3-trifluoromethyl-benzoique ;
- 4-[2-(5,5,8,8-tetramethyl-5,6,7,8-tetrahydro-naphthalene-2-sulfonyl)-ethyl]-benzoate de méthyle ;
- 4-[2-(5,5,8,8-tetramethyl-5,6,7,8-tetrahydro-naphthalene-2-sulfonyl)-ethyl]-benzoate d'éthyle ;
- 4-[2-(5,5,8,8-tetramethyl-5,6,7,8-tetrahydro-naphthalene-2-sulfonyl)-ethyl]-3-trifluoromethyl-benzoate de méthyle;
- 4-[2-(5,5,8,8-tetramethyl-5,6,7,8-tetrahydro-naphthalene-2-sulfonyl)-ethyl]-3-trifluoromethyl-benzoate d'éthyle ;
- acide 2-méthyl-4-[2-(5,5,8,8-tetramethyl-5,6,7,8-tetrahydro-naphthalene-2-sulfonyl)-ethyl]-benzoique ;
- acide 2-fluoro-4-[2-(5,5,8,8-tetramethyl-5,6,7,8-tetrahydro-naphthalene-2-sulfonyl)-ethyl]-benzoique ;
- acide 4-[2-(5,5,8,8-tetramethyl-5,6,7,8-tetrahydro-naphthalene-2-sulfonyl)-ethyl]-2-trifluorométhyl-benzoique ;
- acide 3-methyl-4-[2-(5,5,8,8-tetramethyl-5,6,7,8-tetrahydro-naphtalene-2-sulfonyl)-ethyl]-benzoique ;
- acide 3-fluoro-4-[2-(5,5,8,8-tetramethyl-5,6,7,8-tetrahydro-naphtalene-2-sulfonyl)-ethyl]-benzoique ;
- acide 4-[2-(5,5,8,8-tetramethyl-3-tufluoromethyl-5,6,7,8-tetrahydro-naphtalene-2-sulfonyl)-ethyl]-benzoique ;
- acide 3-methyl-4-[2-(3,5,5,8,8-pentamethyl-5,6,7,8-tetrahydro-naphtalene-2-sulfonyl)-ethyl]-benzoique ;
- acide 2-methyl-4-[2-(3,5,5,8,8-pentamethyl-5,6,7,8-tetrahydro-naphtalene-2-sulfonyl)-ethyl]-benzoique ;
- acide 2,3-dimethyl-4-[2-(5,5,8,8-tetramethyl-5,6,7,8-tetrahydro-naphtalene-2-sulfonyl)-ethyl]-benzoique ;
- acide 2,3-difluoro-4-[2-(5,5,8,8-tetramethyl-5,6,7,8-tetrahydro-naphtalene-2-sulfonyl)-ethyl]-benzoique ; et
- 2-methyl-4-[2-(3,5,5,8,8-pentamethyl-5,6,7,8-tetrahydro-naphtalene-2-sulfonyl)-ethyl]-benzoate de méthyle.

5. Composé selon l'une quelconque des revendications 1 à 4, **caractérisé en ce qu'**il est choisi dans le groupe constitué par :
- acide 4-[2-(5,5,8,8-tetramethyl-5,6,7,8-tetrahydro-naphthalene-2-sulfonyl)-ethyl]-benzoique;
- acide 4-[2-(5,5,8,8-tetramethyl-5,6,7,8-tetrahydro-naphthalene-2-sulfonyl)-ethyl]-3-trifluoromethyl-benzoique; et
- acide 4-[2-(3,5,5,8,8-pentamethyl-5,6,7,8-tetrahydro-naphthalene-2-sulfonyl)-ethyl]-3-trifluoromethyl-benzoique.

6. Composé selon l'une quelconque des revendications 1 à 5, **caractérisé en ce qu'**il est l'acide acide 4-[2-(5,5,8,8-tetramethyl-5,6,7,8-tetrahydro-naphthalene-2-sulfonyl)-ethyl]-benzoique.

7. Composition comprenant un composé selon l'une quelconque des revendications 1 à 6 en association avec un solvant ou un support.

8. Composition cosmétique ou pharmaceutique selon la revendication 7, **caractérisée en ce que** le solvant ou support est dermatologiquement acceptable.

9. Composé selon l'une quelconque des revendications 1 à 6 pour son utilisation en tant que médicament.

10. Composé selon la revendication 9, pour une utilisation pour prévenir ou traiter les pathologies suivantes :
1) les affections dermatologiques liées à un désordre de la kératinisation portant sur la différenciation et sur la prolifération cellulaire ;
2) les troubles de la kératinisation ;
3) les affections dermatologiques avec une composante immuno-allergique inflammatoire, avec ou sans trouble de la prolifération cellulaire ;
4) les désordres cutanés dus à une exposition aux rayonnements U.V. pour réduire les pigmentations et les kératoses actiniques, ou toutes pathologies associées au vieillissement chronologique ou actinique ;
5) Toute condition liée à des proliférations dermiques ou épidermiques bénignes, qu'elles soient ou non d'origine virale ;
6) les dermatoses immunes ;
7) les stigmates de l'atrophie épidermique et/ou dermique induite par les corticostéroïdes locaux ou systémiques, ou toute autre forme d'atrophie cutanée ;
8) les troubles de la cicatrisation, ou pour prévenir ou pour réparer les vergetures, ou encore pour favoriser la cicatrisation ;
9) les affections d'origine fongique au niveau cutané,
10) les désordres de la pigmentation ; et
11) les états cancéreux ou précancéreux, cutanés ou muqueux.

11. Composé selon la revendication 10 pour une utilisation dans le traitement de maladies deimatologiques telles que l'acné, les ichtyoses, les états ichtyosiformes, l'hyperkératose palmoplantaire ou le psoriasis.

12. Composé selon l'une quelconque des revendications 1 à 6 ou composition selon la revendication 7 ou 8 pour une utilisation dans le traitement du vieillissement cutané.

13. Composé ou composition selon la revendication 12 pour une utilisation dans le traitement de l'atrophie cutanée, des taches de pigmentation, de la sécheresse cutanée, de la rugosité cutanée, de l'élastose solaire, et des télangiectasies.

14. Utilisation cosmétique d'un composé selon l'une quelconque des revendications 1 à 6 ou d'une composition selon la revendication 7 ou 8 pour maintenir et/ou stimuler l'hydratation de la peau, pour le traitement des peaux sèches ou pour le traitement ou la prévention du vieillissement de la peau ou des phanères et/ou du photo-vieillissement, pour diminuer la ptôse, ou améliorer la qualité et/ou la transparence de la peau, pour l'amélioration ou la restauration de la souplesse, de l'élasticité et/ou du tonus de la peau, pour le raffermissement de la peau.

15. Composition selon la revendication 7, **caractérisée en ce qu'**elle comprend une dose allant de de 0,000001 % à 10 % en poids total d'un composé de l'invention par rapport au poids total de la composition.

## Patentansprüche

1. Verbindungen oder ein Salz davon mit der folgenden allgemeinen Formel (I): 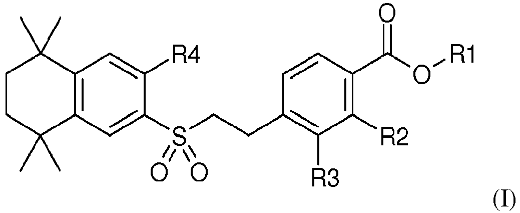 in der:
R1 für ein Wasserstoffatom oder ein C1-C3-Alkyl steht;
R2 für einen Wasserstoff, ein C1-C3-Alkyl, ein Fluor oder einen CF3-Rest steht;
R3 für einen Wasserstoff, ein C1-C3-Alkyl, ein Fluor oder einen CF3-Rest steht;
R4 für einen Wasserstoff, ein C1-C3-Alkyl oder einen CF3-Rest steht.

2. Verbindung gemäß Anspruch 1, **dadurch gekennzeichnet, dass**:
R1 aus der Gruppe ausgewählt ist, bestehend aus einem Wasserstoff, einem Methyl und einem Ethyl;
R2 aus der Gruppe ausgewählt ist, bestehend aus einem Wasserstoff, einem Methyl, einem Fluor oder einem CF3-Rest;
R3 aus der Gruppe ausgewählt ist, bestehend aus einem Wasserstoff, einem Methyl, einem Fluor oder einem CF3-Rest; und
R4 aus der Gruppe ausgewählt ist, bestehend aus einem Wasserstoff, einem Methyl und einem CF3-Rest.

3. Verbindung gemäß Anspruch 1 oder 2, **dadurch gekennzeichnet, dass** R1 für ein Wasserstoffatom steht.

4. Verbindung gemäß einem der Ansprüche 1 bis 3, **dadurch gekennzeichnet, dass** sie aus der Gruppe ausgewählt ist, bestehend aus:
- 4-[2-(5,5,8,8-Tetramethyl-5,6,7,8-tetrahydronaphthalin-2-sulfonyl)-ethyl]-benzoesäure;
- 4-[2-(5,5,8,8-Tetramethyl-5,6,7,8-tetrahydronaphthalin-2-sulfonyl)-ethyl]-3-trifluormethyl-benzoesäure;
- 4-[2-(3,5,5,8,8-Pentamethyl-5,6,7,8-tetrahydronaphthalin-2-sulfonyl)-ethyl]-3-trifluormethyl-benzoesäure;
- 4-[2-(5,5,8,8-Tetramethyl-5,6,7,8-tetrahydronaphthalin-2-sulfonyl)-ethyl]-benzoesäuremethylester;
- 4-[2-(5,5,8,8-Tetramethyl-5,6,7,8-tetrahydronaphthalin-2-sulfonyl)-ethyl]-benzoesäureethylester;
- 4-[2-(5,5,8,8-Tetramethyl-5,6,7,8-tetrahydronaphthalin-2-sulfonyl)-ethyl]-3-tlifluormethyl-benzoesäuremethylester;
- 4-[2-(5,5,8,8-Tetramethyl-5,6,7,8-tetrahydronaphthalin-2-sulfonyl)-ethyl]-3-trifluormethyl-benzoesäureethylester;
- 2-Methyl-4-[2-(5,5,8,8-tetramethyl-5,6,7,8-tetrahydronaphthalin-2-sulfonyl)-ethyl]-benzoesäure;
- 2-Fluor-4-[2-(5,5,8,8-tetramethyl-5,6,7,8-tetrahydronaphthalin-2-sulfonyl)-ethyl]-benzoesäure;
- 4-[2-(5,5,8,8-Tetramethyl-5,6,7,8-tetrahydronaphthalin-2-sulfonyl)-ethyl]-2-trifluormethyl-benzoesäure;
- 3-Methyl-4-[2-(5,5,8,8-tetramethyl-5,6,7,8-tetrahydronaphthalin-2-sulfonyl)-ethyl]-benzoesäure;
- 3-Fluor-4-[2-(5,5,8,8-tetramethyl-5,6,7,8-tetrahydronaphthalin-2-sulfonyl)-ethyl]-benzoesäure;
- 4-[2-(5,5,8,8-Tetramethyl-3-trifluormethyl-5,6,7,8-tetrahydronaphthalin-2-sulfonyl)-ethyl]-benzoesäure;
- 3-Methyl-4-[2-(3,5,5,8,8-pentamethyl-5,6,7,8-tetrahydronaphthalin-2-sulfonyl)-ethyl]-benzoesäure;
- 2-Methyl-4-[2-(3,5,5,8,8-pentamethyl-5,6,7,8-tetrahydronaphthalin-2-sulfonyl)-ethyl]-benzoesäure;
- 2,3-Dimethyl-4-[2-(5,5,8,8-tetramethyl-5,6,7,8-tetrahydronaphthalin-2-sulfonyl)-ethyl]-benzoesäure;
- 2,3-Difluor-4-[2-(5,5,8,8-tetramethyl-5,6,7,8-tetrahydronaphthalin-2-sulfonyl)-ethyl]-benzoesäure; und
- 2-Methyl-4-[2-(3,5,5,8,8-pentamethyl-5,6,7,8-tetrahydronaphthalin-2-sulfonyl)-ethyl]-benzoesäuremethylester.

5. Verbindung gemäß einem der Ansprüche 1 bis 4, **dadurch gekennzeichnet, dass** sie aus der Gruppe ausgewählt ist, bestehend aus:
- 4-[2-(5,5,8,8-Tetramethyl-5,6,7,8-tetrahydronaphthalin-2-sulfonyl)-ethyl]-benzoesäure;
- 4-[2-(5,5,8,8-Tetramethyl-5,6,7,8-tetrahydronaphthalin-2-sulfonyl)-ethyl]-3-trifluormethyl-benzoesäure; und
- 4-[2-(3,5,5,8,8-Pentamethyl-5,6,7,8-tetrahydronaphthalin-2-sulfonyl)-ethyl]-3-trifluormethyl-benzoesäure.

6. Verbindung gemäß einem der Ansprüche 1 bis 5, **dadurch gekennzeichnet, dass** sie 4-[2-(5,5,8,8-Tetramethyl-5,6,7,8-tetrahydronaphthalin-2-sulfonyl)-ethyl]-benzoesäure ist.

7. Zusammensetzung, umfassend eine Verbindung gemäß einem der Ansprüche 1 bis 6 in Kombination mit einem Lösemittel oder einem Träger.

8. Kosmetische oder pharmazeutische Zusammensetzung gemäß Anspruch 7, **dadurch gekennzeichnet, dass** das Lösemittel oder der Träger dermatologisch verträglich ist.

9. Verbindung gemäß einem der Ansprüche 1 bis 6 zur Verwendung als Medikament.

10. Verbindung gemäß Anspruch 9 für eine Verwendung zur Prävention oder Behandlung von folgenden Krankheiten:
1) dermatologischen Erkrankungen, die mit einer Störung der Keratinisation im Zusammenhang mit der Zelldifferenzierung und der Zellproliferation verbunden sind;
2) Störungen der Keratinisation;
3) dermatologischen Erkrankungen mit einer entzündlichen immun-allergischen Komponente, mit oder ohne Störung der Zellproliferation;
4) Hautkrankheiten, die auf eine UV-Strahlenexposition zurückzuführen sind, zur Verringerung der strahleninduzierten Pigmentierungen und Keratosen oder beliebigen Erkrankungen, die mit der chronologischen oder strahleninduzierten Alterung assoziiert sind;
5) jedem beliebigen Zustand, der mit benignen dermalen oder epidermalen Proliferationen verbunden ist, die virusinduziert sein können;
6) Immundermatosen;
7) Stigmata der epidermalen und/oder dermalen Atrophie, die durch lokale oder systemische Corticosteroide induziert wird, oder von einer beliebigen anderen Form der Hautatrophie;
8) Wundheilungsstörungen oder zur Prävention oder Reparatur von Schwangerschaftsstreifen oder auch zur Förderung der Wundheilung;
9) pilzinduzierten Hauterkrankungen;
10) Pigmentierungsstörungen; und
11) präkanzerösen oder kanzerösen Zuständen der Haut oder Schleimhaut.

11. Verbindung gemäß Anspruch 10 für eine Verwendung in der Behandlung von dermatologischen Krankheiten wie beispielsweise Akne, Ichthyosen, ichthyosiformen Zuständen, Hyperkeratosis palmoplantaris oder Psoriasis.

12. Verbindung gemäß einem der Ansprüche 1 bis 6 oder Zusammensetzung gemäß Anspruch 7 oder 8 für eine Verwendung in der Behandlung der Hautalterung.

13. Verbindung oder Zusammensetzung gemäß Anspruch 12 für eine Verwendung in der Behandlung der Hautatrophie, von Pigmentflecken, der trockenen Haut, der rauhen Haut, der Sonnenelastose und von Telangiektasien.

14. Kosmetische Verwendung einer Verbindung gemäß einem der Ansprüche 1 bis 6 oder einer Zusammensetzung gemäß Anspruch 7 oder 8 zur Erhaltung und/oder Stimulierung der Hautfeuchtigkeit, für die Behandlung der trockenen Haut oder für die Behandlung oder Prävention der Alterung der Haut oder Hautanhangsgebilden und/oder der Lichtalterung, zur Verminderung der Ptosis oder zur Verbesserung der Hautqualität und/oder Hautreinheit, für die Verbesserung oder Wiederherstellung der Geschmeidigkeit, der Elastizität und/oder der Spannkraft der Haut, für die Straffung der Haut.

15. Zusammensetzung gemäß Anspruch 7, **dadurch gekennzeichnet, dass** sie eine Dosis von insgesamt 0,000001 Gew.-% bis 10 Gew.-% einer Verbindung der Erfindung bezogen auf das Gesamtgewicht der Zusammensetzung umfasst.

## Claims

1. Compounds or a salt thereof of general formula (I) below: 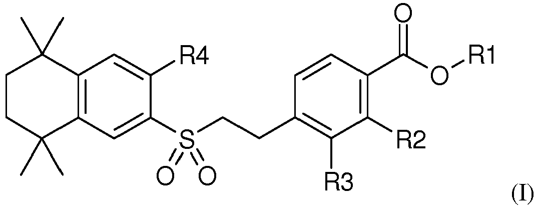 in which:
R1 is a hydrogen atom or a C₁-C₃ alkyl;
R2 is a hydrogen, a C₁-C₃ alkyl, a fluorine, or a CF₃ radical;
R3 is a hydrogen, a C₁-C₃ alkyl, a fluorine, or a CF₃ radical;
R4 is a hydrogen, a C₁-C₃ alkyl, or a CF₃ radical.

2. Compound according to claim 1, **characterized in that**:
R1 is selected from the group consisting of a hydrogen, a methyl and an ethyl;
R2 is selected from the group consisting of a hydrogen, a methyl, a fluorine, or a CF₃ radical;
R3 is selected from the group consisting of a hydrogen, a methyl, a fluorine, or a CF₃ radical; and
R4 is selected from the group consisting of a hydrogen, a methyl and a CF₃ radical.

3. Compound according to claim 1 or 2, **characterized in that** R1 is a hydrogen atom.

4. Compound according to any one of claims 1 to 3, **characterized in that** it is selected from the group consisting of:
- 4-[2-(5,5,8,8-tetramethyl-5,6,7,8-tetrahydro-naphthalene-2-sulfonyl)ethyl]benzoic acid;
- 4-[2-(5,5,8,8-tetramethyl-5,6,7,8-tetrahydro-naphthalene-2-sulfonyl)ethyl]-3-trifluoromethyl-benzoic acid;
- 4-[2-(3,5,5,8,8-pentamethyl-5,6,7,8-tetrahydro-naphthalene-2-sulfonyl)ethyl]-3-trifluoromethyl-benzoic acid;
- methyl 4-[2-(5,5,8,8-tetramethyl-5,6,7,8-tetrahydro-naphthalene-2-sulfonyl)ethyl]benzoate;
- ethyl 4-[2-(5,5,8,8-tetramethyl-5,6,7,8-tetrahydro-naphthalene-2-sulfonyl)ethyl]benzoate;
- methyl 4-[2-(5,5,8,8-tetramethyl-5,6,7,8-tetrahydro-naphthalene-2-sulfonyl)ethyl]-3-trifluoromethyl benzoate;
- ethyl 4-[2-(5,5,8,8-tetramethyl-5,6,7,8-tetrahydro-naphthalene-2-sulfonyl)ethyl]-3-trifluoromethyl benzoate;
- 2-methyl-4-[2-(5,5,8,8-tetramethyl-5,6,7,8-tetrahydro-naphthalene-2-sulfonyl)ethyl]benzoic acid;
- 2-fluoro-4-[2-(5,5,8,8-tetramethyl-5,6,7,8-tetrahydro-naphthalene-2-sulfonyl)ethyl]benzoic acid;
- 4-[2-(5,5,8,8-tetramethyl-5,6,7,8-tetrahydro-naphthalene-2-sulfonyl)ethyl]-2-trifluoromethyl-benzoic acid;
- 3-methyl-4-[2-(5,5,8,8-tetramethyl-5,6,7,8-tetrahydro-naphtalene-2-sulfonyl)ethyl]benzoic acid;
- 3-fluoro-4-[2-(5,5,8,8-tetramethyl-5,6,7,8-tetrahydro-naphtalene-2-sulfonyl)ethyl]benzoic acid;
- 4-[2-(5,5,8,8-tetramethyl-3-trifluoromethyl-5,6,7,8-tetrahydro-naphtalene-2-sulfonyl)ethyl]benzoic acid;
- 3-methyl-4-[2-(3,5,5,8,8-pentamethyl-5,6,7,8-tetrahydro-naphtalene-2-sulfonyl)ethyl]benzoic acid;
- 2-methyl-4-[2-(3,5,5,8,8-pentamethyl-5,6,7,8-tetrahydro-naphtalene-2-sulfonyl)ethyl]benzoic acid;
- 2,3-dimethyl-4-[2-(5,5,8,8-tetramethyl-5,6,7,8-tetrahydro-naphtalene-2-sulfonyl)ethyl]benzoic acid;
- 2,3-difluoro-4-[2-(5,5,8,8-tetramethyl-5,6,7,8-tetrahydro-naphtalene-2-sulfonyl)ethyl]benzoic acid; and
- methyl 2-methyl-4-[2-(3,5,5,8,8-pentamethyl-5,6,7,8-tetrahydro-naphtalene-2-sulfonyl)ethyl]benzoate.

5. Compound according to any one of claims 1 to 4, **characterized in that** it is selected from the group consisting of:
- 4-[2-(5,5,8,8-tetramethyl-5,6,7,8-tetrahydro-naphthalene-2-sulfonyl)ethyl]benzoic acid;
- 4-[2-(5,5,8,8-tetramethyl-5,6,7,8-tetrahydro-naphthalene-2-sulfonyl)ethyl]-3-trifluoromethyl-benzoic acid; and
- 4-[2-(3,5,5,8,8-pentamethyl-5,6,7,8-tetrahydro-naphthalene-2-sulfonyl)ethyl]-3-trifluoromethyl-benzoic acid.

6. Compound according to any one of claims 1 to 5, **characterized in that** it is 4-[2-(5,5,8,8-tetramethyl-5,6,7,8-tetrahydro-naphthalene-2-sulfonyl)ethyl]benzoic acid.

7. Composition comprising a compound according to any one of claims 1 to 6 in combination with a solvent or a support.

8. Cosmetic or pharmaceutical composition according to claim 7, **characterized in that** the solvent or support is dermatologically acceptable.

9. Compound according to any one of claims 1 to 6 for use as a medicinal product.

10. Compound according to claim 9, for use in preventing or treating the following pathologies:
1) dermatological conditions associated with a keratinization disorder relating to cell differentiation and proliferation;
2) keratinization disorders;
3) dermatological conditions with an inflammatory immunoallergic component, with or without a cell proliferation disorder;
4) skin disorders caused by exposure to UV radiation for reducing actinic keratoses and pigmentations, or any pathological conditions associated with chronological or actinic aging;
5) any condition associated with benign dermal or epidermal proliferations, whether or not they are of viral origin;
6) immune dermatoses;
7) stigmata of epidermal and/or dermal atrophy induced by local or systemic corticosteroids, or any other form of cutaneous atrophy;
8) healing disorders, or for treating or repairing stretch marks, or else for promoting healing;
9) conditions of fungal origin at the cutaneous level,
10) pigmentation disorders; and
11) cutaneous or mucosal cancerous or precancerous conditions.

11. Compound according to claim 10 for use in treating dermatological diseases such as acne, ichthyoses, ichthyosiform conditions, palmoplantar hyperkeratosis or psoriasis.

12. Compound according to any one of claims 1 to 6 or composition according to claim 7 or 8 for use in treating skin aging.

13. Compound or composition according to claim 12 for use in treating cutaneous atrophy, pigmentation spots, skin dryness, skin roughness, solar elastosis and telangiectasia.

14. Cosmetic use of a compound according to any one of claims 1 to 6 or a composition according to claim 7 or 8 for maintaining and/or stimulating the moisture of the skin, for treating dry skin or for treating or preventing aging of the skin or skin appendages and/or photoaging, for reducing ptosis, or for improving the quality and/or the transparency of the skin, for improving or restoring the suppleness, the elasticity and/or the tonicity of the skin, for firming the skin.

15. Composition according to claim 7, **characterized in that** it comprises a dose ranging from 0.000001% to 10% by total weight of a compound of the invention relative to the total weight of the composition.
